Europäisches Patentamt

European Patent Office

Office européen des brevets

(11) Veröffentlichungsnummer: **0 228 592**
**A2**

(12) **EUROPÄISCHE PATENTANMELDUNG**

(21) Anmeldenummer: 86116813.6

(22) Anmeldetag: 03.12.86

(51) Int. Cl.⁴: **A 61 L 25/00**

(30) Priorität: 06.12.85 DE 3543164

(43) Veröffentlichungstag der Anmeldung:
15.07.87 Patentblatt 87/29

(84) Benannte Vertragsstaaten:
AT BE CH DE ES FR GB GR IT LI LU NL SE

(71) Anmelder: GMT Gesellschaft für medizinische Technik mbH
Holstenstrasse 2
D-2000 Hamburg 50(DE)

(72) Erfinder: Scherf Helga Prof. Dr.
Farnstrasse 3
D-2000 Hamburg 63(DE)

(72) Erfinder: Lödenkamper Ursula
Weisse Rose 6
D-2000 Hamburg(DE)

(74) Vertreter: Heldt, Gert, Dr. Dipl.-Ing.
Neuer Wall 59 III
D-2000 Hamburg 36(DE)

(54) Knochenzement.

(57) Ein Knochenzement, der über einen großen Zeitraum antibakterielle Substanzen freisetzt, die ein relativ großes Keimspektrum besitzen besteht aus einer Mischung aus mindestens einer antibakteriell wirkenden Substanz und einem unter Beigabe eines Monomers aushärtbaren Kunststoff auf Methacrylbasis. Die antibakteriell wirkende Substanz ist als ein Gyrasehemmer aus der Gruppe der Fluochinolone ausgebildet. Als Gyrasehemmer können Ofloxacine, Pefloxacine, Ciprofloxacine, Enoxacine oder CI 934 Verwendung finden. Der aushärtbare Kunststoff ist als ein mit Zirkondioxyd angereichertes Copolymerpulver ausgebildet.

Die Erfindung betrifft einen Knochenzement bestehend aus einer Mischung aus mindestens einer antibakteriell wirkenden Substanz und einem unter Beigabe eines Monomers aushärtbaren Kunstharz auf Methacrylatbasis.

Ein derartiger Knochenzement hat sich in der Vergangenheit gut bewährt. Allerdings stellte sich heraus, daß die bis jetzt verwendeten antibakteriell wirkenden Substanzen in manchen Fällen von Knocheninfektionen nicht in der Lage waren, das auftretende Keimspektrum zu erfassen. Darüber hinaus ergaben sich in manchen Fällen der verwendeten Antibiotika Schwierigkeiten aufgrund der bei der Aushärtung des Knochenzements auftretenden Reaktionswärme. Bei der Aushärtung des Kunstharzes aufgrund des ihm beigemischten Monomers tritt eine Reaktionswärme auf, die innerhalb des Knochenzements ein Temperaturniveau erreicht, das ausreicht, um manche dem Kunstharz beigemischte Antibiotika in ihrer Wirkung zu beeinträchtigen. In manchen Fällen wurden diese Antibiotika so weitgehend geschädigt, daß sie nicht mehr in der Lage waren, die in einem Entzündungsherd vorhandenen Keime abzutöten.

Aufgabe der vorliegenden Erfindung ist es daher, den Knochenzement der einleitend genannten Art so zu verbessern, daß er in der Lage ist, über einen möglichst großen Zeitraum antibakterielle Substanzen freizusetzen, die ein relativ großes Keimspektrum besitzen.

Diese Aufgabe wird erfindungsgemäß dadurch gelöst, daß die antibakteriell wirkende Substanz als ein Gyrasehemmer aus der Gruppe der Fluochinolone ausgebildet ist.

Ein derartiger Knochenzement besitzt ein relativ großes Keimspektrum, das nicht nur Pseudomonas aeruginosa sondern auch die Enterobacteriaceae und grampositive Kokken umfassen. Die Fluochinolone besitzen einen bakteriziden Wirkungsmechanismus und sind in weitem Umfange temperaturunempfindlich. Es hat sich herausgestellt, daß sie durch die Wärmeentwicklung, die beim Polymerisationsprozeß des Knochenzements ensteht, in ihrer Wirksamkeit nicht beeinträchtigt werden. Darüber hinaus werden sie in den Kunstharz des Knochenzementes so eingebunden, daß sie über eine relativ lange Zeitdauer von beispielsweise zehn Monaten in relativ gleichmäßigen Dosen freigesetzt werden.

Gemäß einer bevorzugten Ausführungsform der Erfindung ist als Gyraschemmer ein Ofloxacin vorgesehen. Dieses Ofloxacin besitzt zwei bakterizide Mechanismen, die auf Keime eines relativ großen Keimspektrums einwirken. Daher ist gerade das Ofloxacin für eine antibakterielle Lokaltherapie besonders gut geeignet.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht.

In den Zeichnungen zeigen:

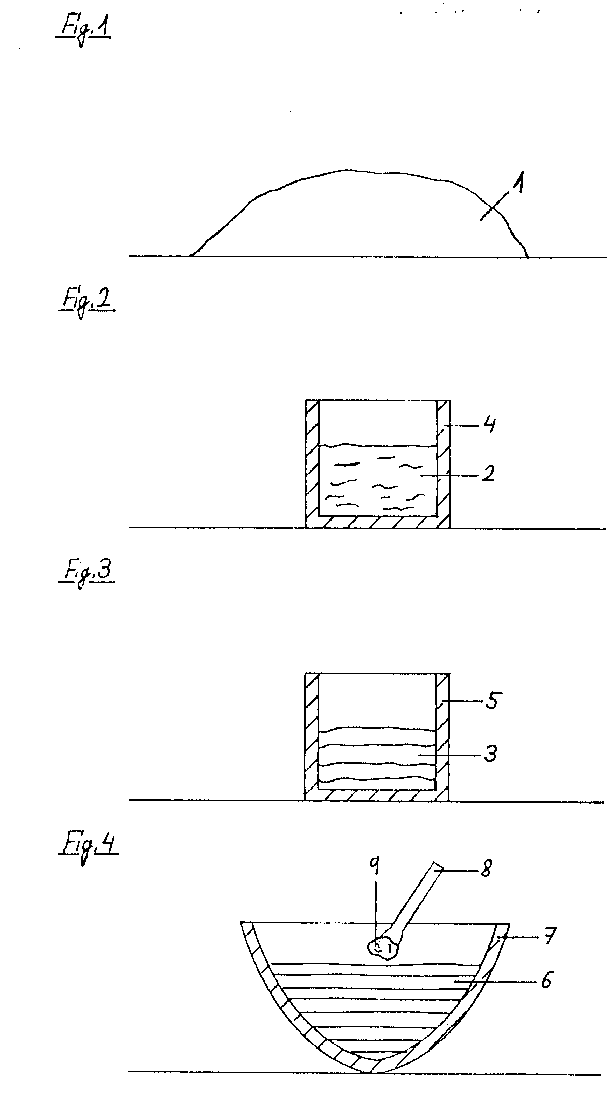

Fig. 1 : eine Ansicht einer bestimmten Menge eines aushärtbaren Kunstharzes zur Herstellung von Knochenzement,

Fig. 2 : eine Seitenansicht eines in einem Gefäß aufbewahrten flüssigen Monomers zur Herstellung von Knochenzement,

Fig. 3 : eine Seitenansicht eines mit einer antibakteriell wirkenden Substanz gefüllten
Gefäßes und

Fig. 4 : eine Seitenansicht eines Mischbehälters,
der zum Teil mit einer teigigen Mischung
gefüllt ist.

Ein Knochenzement besteht im wesentlichen aus einem aushärtbaren Kunstharz 1 auf Methacrylatbasis, einem flüssigen Monomer 2 sowie einer antibakteriell wirkenden Substanz 3. Das flüssige Monomer 2 wird in einem Meßbehälter
4, die antibakteriell wirkende Substanz 3 in einem Meßbehälter 5 abgemessen.

Um eine bestimmte Menge eines Knochenzements 6 herzustellen, wird eine entsprechende Menge des aushärtbaren Kunstharzes 1 in einen Mischbehälter 7 eingefüllt. Die dabei
verwendete Menge des aushärtbaren Kunstharzes 1 entspricht
der für eine bestimmte Zementierungsaufgabe benötigten
Menge. Beispielsweise ist die in den Mischbehälter 7 eingefüllte Menge des aushärtbaren Kunstharzes 1 dafür
vorgesehen, um ein bestimmtes nicht dargestelltes Endogelenk in einen entsprechend vorbereiteten nicht dargestellten Knochen einzusetzen.

Zu dem in den Mischbehälter 7 eingefüllten aushärtbaren
Kunstharz 1 wird die antibakteriell wirkende Substanz 3
hinzugefügt, die im Meßbehälter 5 abgemessen worden ist.
Dabei erfolgt die Abmessung der antibakteriell wirkenden
Substanz 3 entsprechend der Menge des benötigten Kunstharzes 1. Dabei wird einem als Kunstharz 1 verwendeten
Copolymerpulver ein Ofloxacin als Gyrasehemmer im Verhältnis von 1o:1 zugemischt.

0228592

Nachdem das Copolymerpulver und das Ofloxacin mit Hilfe eines Spatels 8 kräftig durchmischt worden sind, wird dem Gemisch das im Meßbehälter 4 abgemessene flüssige Monomer 2 beigegeben. Die Menge des flüssigen Monomers 2 wird dabei so bemessen, daß nach intensiver Durchmischung des mit der antibakteriell wirkenden Substanz durchmischten Kunstharzes 1 mit dem flüssigen Monomer 2 eine teigige Masse 9 entsteht, die ohne Schwierigkeit in einen nicht dargestellten Knochen eingebracht werden kann. Zur Herstellung dieser teigigen Masse 9 wird das aus dem Kunstharz 1, der antibakteriell wirkenden Substanz 3 sowie dem flüssigen Monomer 2 bestehende Gemisch mit dem Spatel 8 kräftig durchmischt, bis sich die teigige Masse 9 ausbildet. Dabei wird im Regelfall ein Mischungsverhältnis von aushärtbarem Kunstharz 1 zu flüssigem Monomer 2 wie 2:1 eingehalten. Beispielsweise wird einer Menge von 4o g aushärtbarem Kunstharz 2o ml flüssiges Monomer hinzugefügt.

Zweckmäßigerweise wird dem aushärtbaren Kunstharz 1 vor der Zugabe der antibakteriell wirkenden Substanz 3 ein Zirkondioxyd hinzugemischt. Dieses Zirkondioxyd bewirkt eine gleichmäßige Freisetzung der antibakteriellen Substanz aus dem in den Knochen eingebrachten und inzwischen ausgehärteten Knochenzement.

Statt des Ofloxacin können auch andere Fluochinolone als Gyrasehemmer eingesetzt werden. Beispielsweise ist es möglich, statt des Ofloxacin auch Pefloxacin, Ciprofloxacin, Enoxacin oder CI 934 zu verwenden.

Unabhängig von der Art des verwendeten Gyrasehemmers werden alle der genannten Substanzen in Pulverform mit dem ebenfalls in Pulverform vorliegenden Kunstharz 1 vermischt. Dabei werden je nach dem jeweils beabsichtigten Einsatzzweck feste Mischungsverhältnisse eingehalten, die sich im Verhältnis des Gyrasehemmers zum Copolymerpulver zwischen 1:1o und 1:2o bewegen. Für Therapiezwecke hat sich das Mischungsverhältnis 1:2o als besonders wirkungsvoll erwiesen.

Nachdem die Pulvermischung aus Gyrasehemmer und Kunstharz 1 während einiger Minuten kräftig durchmischt worden ist, wird das Monomer 2 in flüssiger Form dem Gemisch beigegeben. Durch die Beigabe entsteht ein teigiger Knochenzement, bei dem dem als Copolymerpulver vorgesehenen Kunstharz 1 das flüssige Monomer 2 im Verhältnis 2:1 beigemischt wird. Beispielsweise werden 4o g Copolymerpulver 2o ml flüssiges Monomer hinzugefügt. Die auf diese Weise erhaltene teigige Masse enthält von der antibakteriell wirkenden Substanz 3 je nach Verwendungszweck des Knochenzements 2 g, 3 g oder 4 g. Der auf diese Weise erhaltene Knochenzement besitzt eine hohe Wirkstoffkonzentration am Ort der Infektion und ist daher zu einer antibakteriellen Lokaltherapie gut geeignet.

Patentansprüche:

1. Knochenzement bestehend aus einer Mischung aus mindestens einer antibakteriell wirkenden Substanz und einem unter Beigabe eines Monomers aushärtbaren Kunstharz auf Methacrylatbasis, dadurch gekennzeichnet, daß die antibakteriell wirkende Substanz (3) als ein Gyrasehemmer aus der Gruppe der Fluochinolone ausgebildet ist.

2. Knochenzement nach Anspruch 1, dadurch gekennzeichnet, daß als Gyrasehemmer ein Ofloxacin vorgesehen ist.

3. Knochenzement nach Anspruch 1, dadurch gekennzeichnet, daß als Gyrasehemmer ein Pefloxacin vorgesehen ist.

4. Knochenzement nach Anspruch 1, dadurch gekennzeichnet, daß als Gyrasehemmer ein Ciprofloxacin vorgesehen ist.

5. Knochenzement nach Anspruch 1, dadurch gekennzeichnet, daß als Gyrasehemmer ein Enoxacin vorgesehen ist.

6. Knochenzement nach Anspruch 1, dadurch gekennzeichnet, daß als Gyrasehemmer CI 934 vorgesehen ist.

7. Knochenzement nach Anspruch 1 bis 6, dadurch gekennzeichnet, daß als aushärtbarer Kunstharz (1) ein Copolymerpulver vorgesehen ist.

8. Knochenzement nach Anspruch 1 bis 7, dadurch gekennzeichnet, daß als aushärtbarer Kunstharz (1) ein mit Zirkondioxyd angereichertes Copolymerpulver vorgesehen ist.

9. Knochenzement nach Anspruch 1 bis 8, dadurch gekennzeichnet, daß der Gyrasehemmer dem Copolymerpulver in Pulverform beigemischt ist.

10. Knochenzement nach Anspruch 1 bis 9, dadurch gekennzeichnet, daß der Gyrasehemmer dem Copolymerpulver in einem festen Verhältnis beigemischt ist, das zwischen 1:1o und 1:2o liegt.

11. Knochenzement nach Anspruch 1 bis 1o, dadurch gekennzeichnet, daß der Gyrasehemmer dem Copolymerpulver zu Therapiezwecken im Verhältnis 1:2o beigemischt ist.

12. Knochenzement nach Anspruch 1 bis 11, dadurch gekennzeichnet, daß dem mit dem Gyrasehemmer vermischten Copolymerpulver das Monomer (2) in flüssiger Form beigemischt ist.

13. Knochenzement nach Anspruch 1 bis 12, dadurch gekennzeichnet, daß das mit dem Gyrasehemmer vermischte Copolmyerpulver nach Zugabe des flüssigen Monomers (2) einen Teig bildet.

14. Knochenzement nach Anspruch 1 bis 13, dadurch gekennzeichnet, daß das Copolymerpulver mit dem flüssigen Monomer (2) im Verhältnis von 2:1 steht.

_Fig.1_

_Fig.2_

_Fig.3_

_Fig.4_